# EUROPEAN PATENT APPLICATION

(11) **EP 3 872 181 A1**
(43) Date of publication of application: **01.09.2021**
(21) Application number: 19877208.9
(22) Date of filing: 24.10.2019
(51) Int. Cl.: C12N 15/54, A61K 35/17, A61K 45/00, A61P 35/00, A61P 43/00, C12N 5/0783, C12N 5/10, C12Q 1/48, G01N 33/15, G01N 33/50, C12N 9/10

(54) **USE OF GLUCOSYLCERAMIDE SYNTHASE GENE-DEFICIENT T CELL AND THERAPEUTIC UTILIZATION THEREOF**

(30) Priority: 25.10.2018 JP 2018200480
(71) Applicant: School Corporation, Azabu Veterinary Medicine Educational Institution, Sagamihara-shi, Kanagawa 252-5201 (JP); Tella, Inc., Tokyo 160-0023 (JP)
(72) Inventor: YAMASHITA, Tadashi, Kanagawa 252-5201 (JP); NAGANE, Masaki, Kanagawa 252-5201 (JP); IKEDA, Teruo, Kanagawa 252-5201 (JP); MIYATAKE, Shoichiro, Kanagawa 252-5201 (JP); OKAMOTO, Mariko, Kanagawa 252-5201 (JP)
(74) Representative: Vossius & Partner Patentanwälte Rechtsanwälte mbB
(86) International application number: PCT/JP2019/041627
(87) International publication number: WO 2020/085414

(57) **Abstract**

The prevent invention provides an activated T cell in which the expression of an immune checkpoint molecule is suppressed, a method for producing the T cell, and a method for screening for a substance that can be used in the production of the T cell. Specifically, the prevent invention includes, as solving means, a method for producing or inducing a T cell in which the expression of an immune checkpoint molecule is not induced, the method comprising reducing or losing the function of UDP-glucose ceramide glucosyltransferase (UGCG) or ganglioside in a T cell, and a T cell produced or induced by the aforementioned method.

SEQUENCE LISTING

<110>  School Corporation, Azabu Veterinary Medicine Educational
       Institution

<120>  Use of UGCG-deficient T cell and therapeutic use of the T cell

<130>  TPC0297AZU

<150>  2018-200480
<151>  2018-10-25

<160>  2

<170>  PatentIn version 3.5

<210>  1
<211>  394
<212>  PRT
<213>  Homo sapiens

<400>  1

Met Ala Leu Leu Asp Leu Ala Leu Glu Gly Met Ala Val Phe Gly Phe
1               5                   10                  15

Val Leu Phe Leu Val Leu Trp Leu Met His Phe Met Ala Ile Ile Tyr
            20                  25                  30

Thr Arg Leu His Leu Asn Lys Lys Ala Thr Asp Lys Gln Pro Tyr Ser
        35                  40                  45

Lys Leu Pro Gly Val Ser Leu Leu Lys Pro Leu Lys Gly Val Asp Pro
    50                  55                  60

Asn Leu Ile Asn Asn Leu Glu Thr Phe Phe Glu Leu Asp Tyr Pro Lys
65                  70                  75                  80

Tyr Glu Val Leu Leu Cys Val Gln Asp His Asp Asp Pro Ala Ile Asp
                85                  90                  95

Val Cys Lys Lys Leu Leu Gly Lys Tyr Pro Asn Val Asp Ala Arg Leu
            100                 105                 110

Phe Ile Gly Gly Lys Lys Val Gly Ile Asn Pro Lys Ile Asn Asn Leu
        115                 120                 125

Met Pro Gly Tyr Glu Val Ala Lys Tyr Asp Leu Ile Trp Ile Cys Asp
    130                 135                 140

Ser Gly Ile Arg Val Ile Pro Asp Thr Leu Thr Asp Met Val Asn Gln
145                 150                 155                 160

Met Thr Glu Lys Val Gly Leu Val His Gly Leu Pro Tyr Val Ala Asp
                165                 170                 175

Arg Gln Gly Phe Ala Ala Thr Leu Glu Gln Val Tyr Phe Gly Thr Ser
            180                 185                 190

His Pro Arg Tyr Tyr Ile Ser Ala Asn Val Thr Gly Phe Lys Cys Val
        195                 200                 205

Thr Gly Met Ser Cys Leu Met Arg Lys Asp Val Leu Asp Gln Ala Gly
    210                 215                 220

Gly Leu Ile Ala Phe Ala Gln Tyr Ile Ala Glu Asp Tyr Phe Met Ala
225                 230                 235                 240

Lys Ala Ile Ala Asp Arg Gly Trp Arg Phe Ala Met Ser Thr Gln Val
                245                 250                 255

Ala Met Gln Asn Ser Gly Ser Tyr Ser Ile Ser Gln Phe Gln Ser Arg
            260                 265                 270

Met Ile Arg Trp Thr Lys Leu Arg Ile Asn Met Leu Pro Ala Thr Ile
        275                 280                 285

Ile Cys Glu Pro Ile Ser Glu Cys Phe Val Ala Ser Leu Ile Ile Gly
    290                 295                 300

Trp Ala Ala His His Val Phe Arg Trp Asp Ile Met Val Phe Phe Met
305                 310                 315                 320

Cys His Cys Leu Ala Trp Phe Ile Phe Asp Tyr Ile Gln Leu Arg Gly
                325                 330                 335

Val Gln Gly Gly Thr Leu Cys Phe Ser Lys Leu Asp Tyr Ala Val Ala
            340                 345                 350

Trp Phe Ile Arg Glu Ser Met Thr Ile Tyr Ile Phe Leu Ser Ala Leu
        355                 360                 365

Trp Asp Pro Thr Ile Ser Trp Arg Thr Gly Arg Tyr Arg Leu Arg Cys
    370                 375                 380

Gly Gly Thr Ala Glu Glu Ile Leu Asp Val
385                 390

<210>  2
<211>  1185
<212>  DNA

<213>  Homo sapiens

<400>  2
atggcgctgc tggacctggc cttggaggga atggccgtct tcgggttcgt cctcttcttg      60

gtgctgtggc tgatgcattt catggctatc atctacaccc gattacacct caacaagaag     120

gcaactgaca aacagcctta tagcaagctc ccaggtgtct ctcttctgaa accactgaaa     180

ggggtagatc ctaacttaat caacaacctg gaaacattct ttgaattgga ttatcccaaa     240

tatgaagtgc tcctttgtgt acaagatcat gatgatccag ccattgatgt atgtaagaag     300

cttcttggaa aatatccaaa tgttgatgct agattgttta taggtggcaa aaaagttggc     360

attaatccta aaattaataa tttaatgcca ggatatgaag ttgcaaagta tgatcttata     420

tggatttgtg atagtggaat aagagtaatt ccagatacgc ttactgacat ggtgaatcaa     480

atgacagaaa aagtaggctt ggttcacggg ctgccttacg tagcagacag acagggcttt     540

gctgccacct tagagcaggt atattttgga acttcacatc caagatacta tatctctgcc     600

aatgtaactg gtttcaaatg tgtgacagga atgtcttgtt taatgagaaa agatgtgttg     660

gatcaagcag gaggacttat agcttttgct cagtacattg ccgaagatta ctttatggcc     720

aaagcgatag ctgaccgagg ttggaggttt gcaatgtcca ctcaagttgc aatgcaaaac     780

tctggctcat attcaatttc tcagtttcaa tccagaatga tcaggtggac caaactacga     840

attaacatgc ttcctgctac aataatttgt gagccaattt cagaatgctt tgttgccagt     900

ttaattattg gatgggcagc ccaccatgtg ttcagatggg atattatggt atttttcatg     960

tgtcattgcc tggcatggtt tatatttgac tacattcaac tcaggggtgt ccagggtggc     1020

acactgtgtt tttcaaaact tgattatgca gtcgcctggt tcatccgcga atccatgaca    1080

atatacattt ttttgtctgc attatgggac ccaactataa gctggagaac tggtcgctac    1140

agattacgct gtgggggtac agcagaggaa atcctagatg tataa                    1185

## Description

### Technical Field

The present invention relates to use of a T cell, in which the function of UDP-glucose ceramide glucosyltransferase is reduced or lost, and the like.

### Background Art

At present, surgical therapy such as surgery, drug therapy using anticancer agents, radiation therapy, and immunotherapy using immune checkpoint inhibitors and the like, have been carried out as cancer therapies. In recent years, among these therapies, the immunotherapy using immune checkpoint inhibitors has attracted attention as a treatment method comprising inhibiting the binding between an immune checkpoint molecule and a ligand thereof to suppress immunosuppressive signaling and to release suppression of T cell activation.

To date, various immune checkpoint molecules have been identified, and examples of reported representative immune checkpoint molecules may include CTLA-4 (cytotoxic T-lymphocyte associated antigen-4) and PD-1 (programmed cell death-1/CD279). Activation of T cells is induced by antigen stimulation mediated by a T-cell receptor (TCR) and the binding between CD28 on the T cell and B7 (CD80/CD86) on an antigen-presenting cell. However, on an activated T cell, CTLA-4 having higher affinity for B7 than CD28 is expressed. When B7 binds to this CTLA-4, signals mediated by CD28 are blocked and activation of the T cell is suppressed (Non Patent Literature 1). Moreover, it has been known that PD-1 is expressed on an activated T cell, and that when the ligands thereof, such as PD-L1 and PD-L2, bind to PD-1, T cell activation thereof is suppressed. PD-L1 and PD-L2 are expressed on cancer cells, and when these molecules bind to activated T cells, activation of the T cells is suppressed (Non Patent Literature 1).

Thus, cancer cells avoid attack by the immune system through inactivation of T cells. However, it has been known that immune checkpoint blockade using an antibody having a long half-life in blood is likely to generate T cells reacting with self-antigens, other than tumor cells, and that it would cause severe side effects such as pneumonia and pancreatitis. Hence, a method of activating T cells that specifically recognize tumor cells currently attracts attention. Examples of the treatment method using such activated T lymphocytes may include CAT (CD3-activated T lymphocyte) therapy and CAR-T (chimeric antigen receptor T cell) therapy. This treatment method is a method comprising allowing subject's own CD3-activated T lymphocytes to proliferate *in vitro* according to various means, and then returning the thus proliferating T lymphocytes into the body, so as to enhance the aggressiveness of the T lymphocytes against cancer cells. Not only T lymphocytes, but also activated T cells are considered to be effective as a means of attacking cancer cells. However, it has been known that these methods are also suppressed *in vivo* by an immune checkpoint mechanism of using PD-1 or the like. In view of the foregoing, it has been desired to develop a method for producing a T cell that specifically recognizes a tumor cell and has low sensitivity to the immune checkpoint.

Glycosphingolipid (GSL) as one type of membrane constituent molecule consists of lipid and sugar chain moieties. GSL is localized in a lipid raft of a cell membrane, and plays an important role in various cell processes including activation of T cells through T-cell receptor (TCR) signals (Non Patent Literature 2). Hence, it is considered that a novel cancer immunotherapy that is similar to or different from the aforementioned immune checkpoint inhibitor therapy or CAT therapy can be established by elucidating the function of glycosphingolipids in activation of T cells. It has been reported so far that a UDP-glucose ceramide glucosyltransferase (UGCG) inhibitor, 1-phenyl-2-hexadecanoylamino-3-morpholino-1-propanol (PPMP) synergistically enhances the cytotoxicity of fenretinide (4-hydroxyphenylretinamide: 4-HPR) on a neuroblastoma cell line and an acute leukemia-derived cell line ALL (Patent Literature 1).

Nevertheless, at the present moment, the correlation between cancer immunity associated with T cells and glycosphingolipids has not yet been elucidated in many respects.

### Citation List

### Patent Literature

Patent Literature 1: WO2005/049827

### Non Patent Literature

Non Patent Literature 1: Pardoll, Nat Rev Cancer 12: 252-264, 2012
Non Patent Literature 2: Nagafuku et al., Proc Natl Acad Sci U S A. 109(6): E336-42. doi: 10.1073/pnas.1114965109. 2012

### Summary of Invention

### Technical Problem

Under the above-described circumstances, it is an object of the present invention to provide an activated T cell in which the expression of an immune checkpoint molecule is suppressed, a method for producing the T cell, and a method for screening for a substance that can be used to produce the T cell.

### Solution to Problem

Focusing on glucosylceramide as a precursor of sphingolipid, the present inventors have transplanted a tumor into a mouse in which UDP-glucose ceramide glucosyltransferase (UGCG) that synthesizes glucosylceramide from ceramide was deleted in a T cell-specific manner (i.e., a T cell-specific UGCG gene-deficient mouse), and thereafter, the present inventors have studied regarding the influence on tumor growth and the properties of the UGCG gene-deficient T cells derived from the mouse.

As a result, it was revealed that tumor growth is retarded and the survival period is prolonged in the T cell-specific UGCG gene-deficient mouse into which a tumor has been transplanted, and that the ratio of CD3⁺/CD8⁺ T cells is significantly increased in the spleen derived from the T cell-specific UGCG gene-deficient mouse after the tumor transplantation. Moreover, when wild-type mouse spleen-derived T cells were activated *ex vivo,* the expression level of PD-1 as an immune checkpoint molecule was increased by a negative feedback to the stimulation. On the other hand, even if T cells derived from the spleen of a T cell-specific UGCG gene-deficient mouse were activated *ex vivo,* an increase in the expression level of PD-1 was not observed.

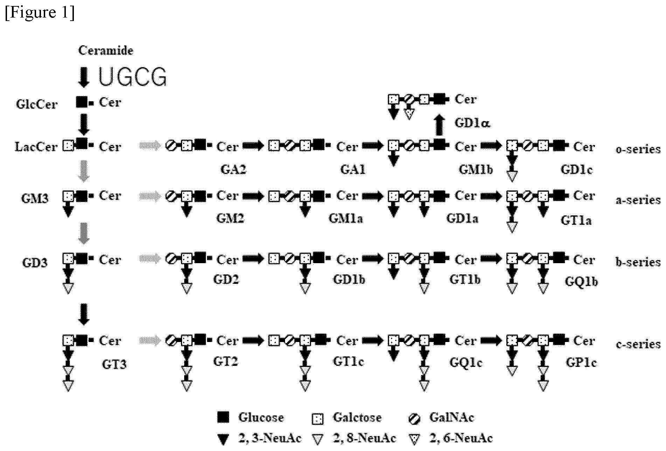

Besides, UGCG is a synthase located most upstream of a synthetic pathway of gangliosides (i.e., glycosphingolipids with sialic acids linked on the sugar chain moieties thereof), and T cells deleting UGCG become T cells not having any types of gangliosides (Figure 1). It is considered that the synthesis of individual gangliosides is inhibited, for example, using a GM3 synthesis inhibitor, etc. However, since UGCG is located most upstream of the synthetic pathway, it can efficiently inhibit the biosynthesis of all gangliosides, and thus, it is anticipated that various expressed physiological activities will be maximized by inhibition of the biosynthesis of gangliosides.

The present invention has been completed based on the aforementioned findings.

Specifically, the present invention includes the following (1) to (13).
(1) A method for producing or inducing a T cell in which the expression of an immune checkpoint molecule is not induced, the method comprising reducing or losing the function of UDP-glucose ceramide glucosyltransferase (UGCG) or ganglioside in a T cell.
(2) The method according to the above (1), which is characterized in that the immune checkpoint molecule is PD-1, TIM3 (T-cell Immunoglobulin and Mucin domain 3), or CTLA4.
(3) The method according to the above (1) or (2), wherein the method of reducing or losing the function of the UGCG is a reduction in the expression of the UGCG gene or a loss thereof.
(4) The method according to the above (1) or (2), which is characterized in that the method of reducing or losing the function of the UGCG is a treatment with a UDP-glucose ceramide glucosyltransferase inhibitor.
(5) A T cell comprising UGCG whose function is reduced or lost, in which an increase in the expression of an immune checkpoint molecule by activation is not induced.
(6) An immune checkpoint inhibitor comprising a UGCG inhibitor as an active ingredient.
(7) The inhibitor according to the above (6), which is characterized in that it is supported on a drug carrier that targets a T cell.
(8) A pharmaceutical composition for the prevention or treatment of a cancer, comprising the T cell according to the above (5) or the inhibitor according to the above (6) or (7).
(9) An agent for suppressing the expression of PD-1, TIM3, and/or CTLA4 in a T cell, the agent comprising a UGCG inhibitor as an active ingredient.
(10) An agent for maintaining the activation of a T cell, the agent comprising a UGCG inhibitor as an active ingredient.
(11) A method for screening for an immune checkpoint-inhibiting substance, comprising allowing a test substance to come into contact with a T cell, and then measuring the amount of Glc-Cer generated in the T cell.
(12) A method for screening for an immune checkpoint-inhibiting substance, comprising allowing a test substance to come into contact with a T cell, and then measuring the expression level of UGCG in the T cell.
(13) The screening method according to the above (11) or the above (12), wherein the immune checkpoint-inhibiting substance is a preventive or therapeutic agent for cancer.

### Advantageous Effects of Invention

According to the present invention, it becomes possible to provide a T cell that can be used in cancer immunotherapies. In addition, it becomes possible to develop a novel cancer immunotherapy by using the T cell.

### Brief Description of Drawings

[Figure 1] Figure 1 shows a synthetic pathway of ganglioside.

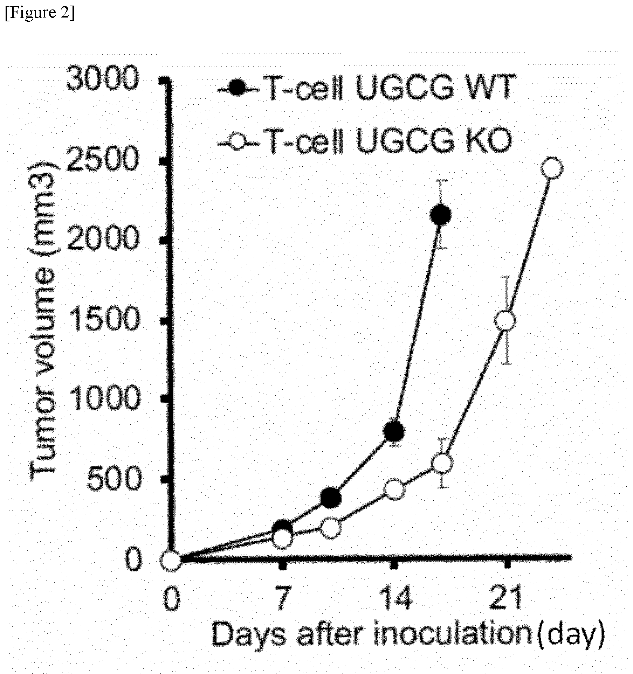

[Figure 2] Figure 2 shows a change in the volume of a tumor transplanted into a T cell-specific UGCG gene-deficient mouse. "T-cell UGCG WT" and "T-cell UGCG KO" indicate control mice and T cell-specific UGCG gene-deficient mice, respectively. In each mouse group, n = 8.

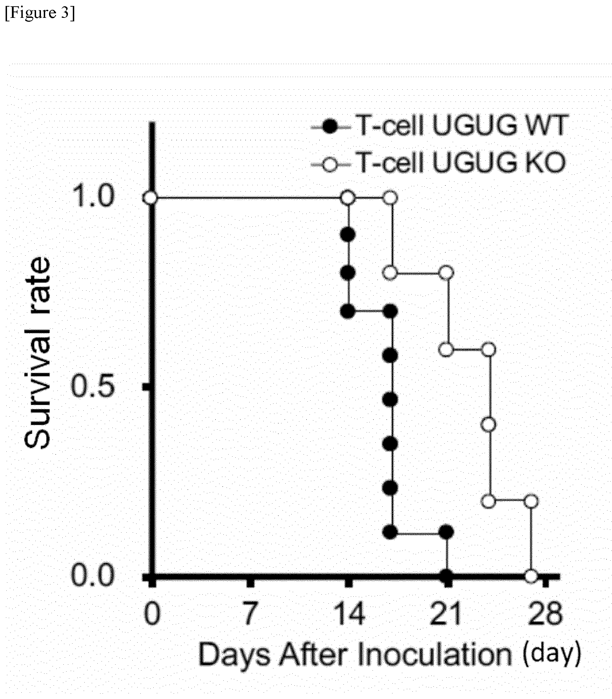

[Figure 3] Figure 3 shows the survival rate of a T cell-specific UGCG gene-deficient mouse, into which mouse B16F10 melanoma cells were transplanted. "T-cell UGCG WT" and "T-cell UGCG KO" indicate control mice and T cell-specific UGCG gene-deficient mice, respectively. In each mouse group, n = 8.

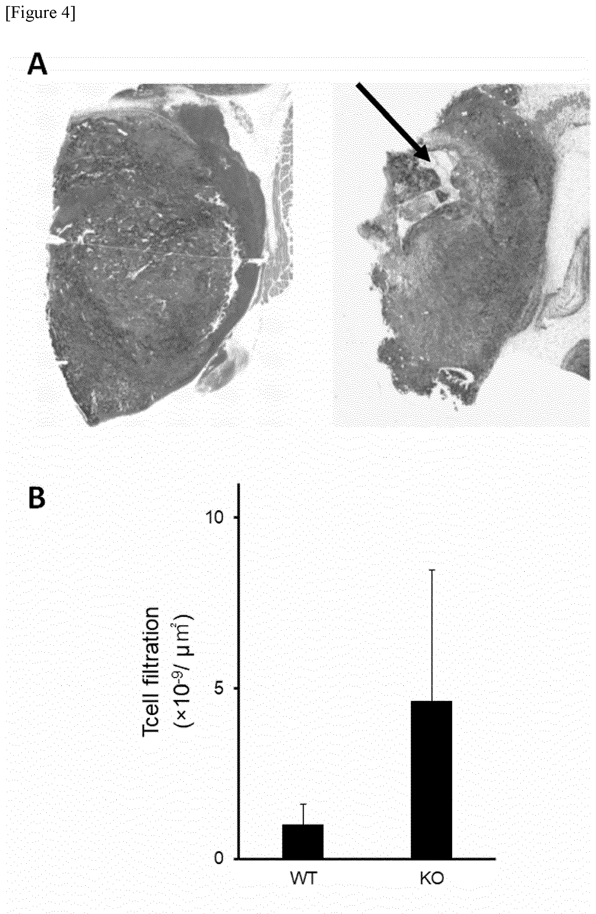

[Figure 4] Figure 4 shows observation of the tumor tissues of a T cell-specific UGCG gene-deficient mouse (KO) and a control mouse (WT). Figure 4(A) shows the HE staining results of the tumor tissues of a T cell-specific UGCG gene-deficient mouse (right) and a control mouse (left). The arrow shows T cells observed around the tumor tissues of the T cell-specific UGCG gene-deficient mouse. Figure 4B shows the results obtained by counting the number of T cells present in the tumor tissues of Figure 3A (right) by visual observation.

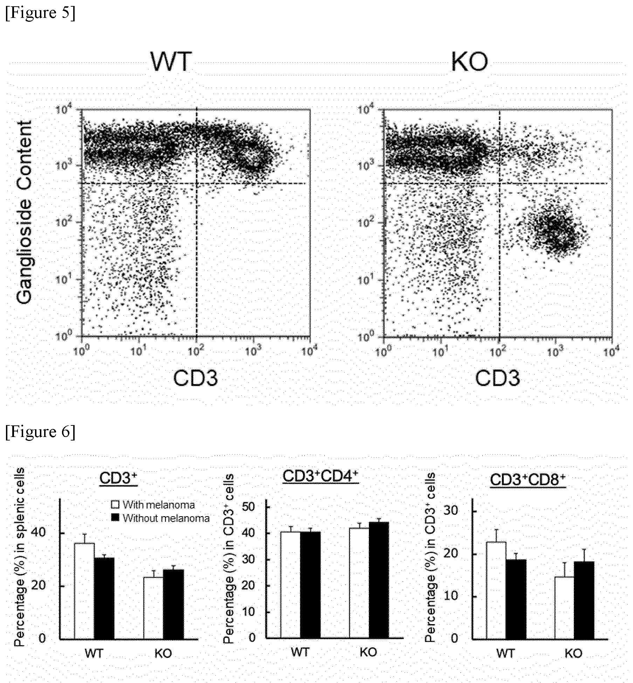

[Figure 5] Figure 5 shows the results obtained by analyzing gangliosides in T cells in the spleen of a T cell-specific UGCG gene-deficient mouse, using a flow cytometer. "WT" and "KO" indicate the results of a control mouse and the results of a T cell-specific UGCG gene-deficient mouse, respectively.
[Figure 6] Figure 6 shows the results of analyzing the configuration of T cells in the spleen of T cell-specific UGCG gene-deficient mice. "WT" and "KO" indicate control mice and T cell-specific UGCG gene-deficient mice, respectively. In each mouse group, n = 8.

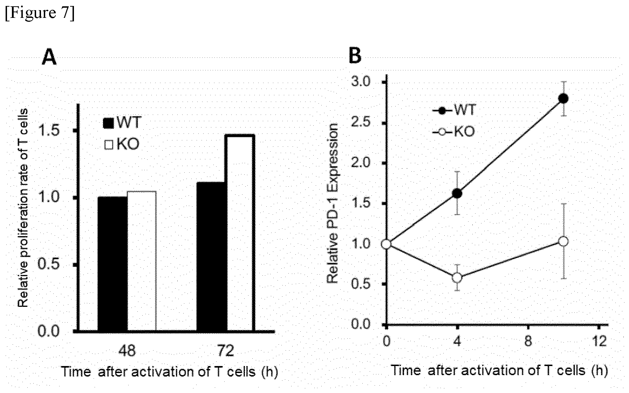

[Figure 7] Figure 7 shows the analysis of activated T cells derived from T cell-specific UGCG gene-deficient mice. Figure 7A shows the results obtained by activating T cells prepared from the spleen, and then measuring the proliferation rate of individual T cells at 48 hours and 72 hours after the T cell activation. Figure 7B shows the results obtained by activating T cells prepared from the spleen, and then measuring the expression level of a PD-1 gene over time. "WT" and "KO" indicate control mice and T cell-specific UGCG gene-deficient mice, respectively. In each mouse group, n = 4.

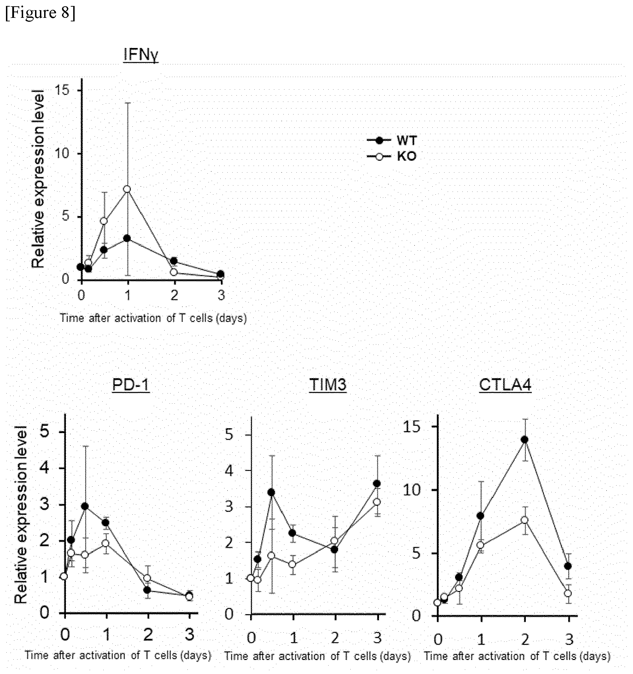

[Figure 8] Figure 8 shows the analysis of activated CD-positive T cells derived from T cell-specific UGCG gene-deficient mice. The figure shows the results obtained by activating CD4-positive T cells prepared from the spleen, and then measuring the expression levels of IFNy, PD-1, TIM3 and CTLA4 genes over time. "WT" and "KO" indicate control mice and T cell-specific UGCG gene-deficient mice, respectively. In each mouse group, n = 3.

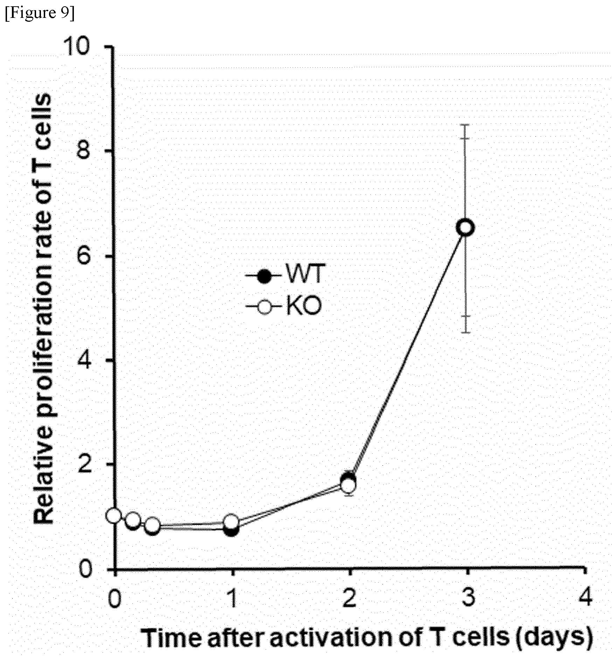

[Figure 9] Figure 9 shows the measurement of the proliferation rate of activated T cells derived from T cell-specific UGCG gene-deficient mice. The figure shows the results obtained by activating T cells prepared from the spleen, and then measuring the proliferation rate thereof over time. "WT" and "KO" indicate control mice and T cell-specific UGCG gene-deficient mice, respectively. In each mouse group, n = 3.

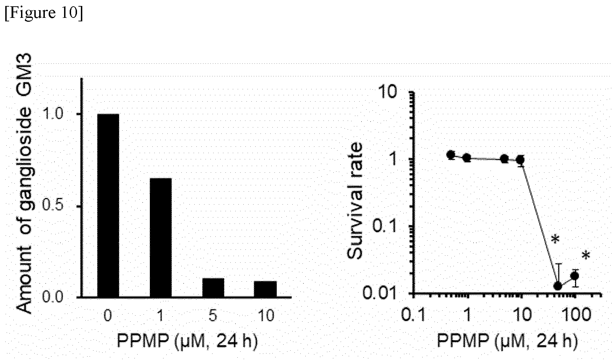

[Figure 10] Figure 10 shows the studies regarding the influence of a PPMP treatment performed *in vitro* on T cells. The isolated T cells were treated with PPMP in concentrations of 0, 1, 5 and 10 µM for 24 hours, and then measuring the expression level of GM3 (left view) and the proliferation rate of the cells (right view).

### Description of Embodiments

A first embodiment of the present invention relates to a method for producing or inducing a T cell in which the expression of an immune checkpoint molecule is not induced, the method comprising reducing or losing the function of UDP-glucose ceramide glucosyltransferase (UGCG) or ganglioside in a T cell.

The present inventors have found that, even if T cells derived from the spleen of a T cell-specific UGCG gene-deficient mouse, namely, UGCG gene-deficient T cells are activated (by co-stimulation with TCR/CD3 and CD28), the expression of immune checkpoint molecules, PD-1, TIM3 and CTLA4, is not induced. Since immune checkpoint molecules such as PD-1 are not induced in UGCG gene-deficient T cells that have undergone activating stimulus, it is assumed that the activated state of the T cells is sustained, and that the immune response of the T cells to cancer cells is also sustained. In fact, it was confirmed that the tumor volume of a melanoma transplanted into such a T cell-specific UGCG gene-deficient mouse increases at a volume-increasing speed that is slower than in the case of the tumor volume of a melanoma transplanted into a wild-type mouse, and that the survival period of the T cell-specific UGCG gene-deficient mouse, into which the tumor has been transplanted, is prolonged. Accordingly, T cells produced by the method according to the first embodiment (hereinafter also referred to as "the method for producing T cells of the present invention") can be used to enhance an immune response to cancer cells.

Moreover, UGCG is located most upstream of a ganglioside synthetic pathway, and thus, if the activity of this enzyme is inhibited, the biosynthesis of all gangliosides located downstream of the synthetic pathway is inhibited. Taking into consideration this respect, it is considered that the same effects as those in the case of reducing or suppressing the function of UGCG can be obtained even by reducing or suppressing the function of one or more gangliosides that are synthesized downstream of the synthetic pathway, namely, by reducing or suppressing, for example, the biosynthesis of the gangliosides.

In the first embodiment, the method of "reducing or losing a ganglioside(s) may include inhibition or suppression of any one or more ganglioside synthetic pathways. More specifically, the activity of one or more enzymes that catalyze each process of synthesizing a ganglioside shown in Figure 1 is inhibited or suppressed, so that the biosynthesis of each ganglioside is inhibited or suppressed. As a result, a reduction or a loss in the function of the ganglioside(s) provoked (i.e., the amounts of the ganglioside(s) expressed in cells are reduced, or the ganglioside(s) disappear). Inhibition of the enzyme activity can be achieved by addition of an enzyme activity inhibitor to cells (wherein the inhibitor may be a compound, or may also be a neutralizing antibody). Even if an appropriate inhibitor is not found, inhibition of the enzyme activity can also be achieved by inhibiting or suppressing the expression of the enzyme gene, for example, according to RNA interference (a method of using siRNA, shRNA or the like). Besides, nucleic acids inhibiting or suppressing the expression of the above-described enzyme gene, such as RNAs, are also included in the present inhibitor.

In the case of using the RNA interference method, for example, the synthetic process of LacCer → GM3 can be inhibited or suppressed by RNA interference of an ST3GAL5 gene, the synthetic process of GM3 → GD3 can be inhibited or suppressed by RNA interference of an ST8SIA1 gene, and the synthetic processes of GM3 → GM2 and GD3 → GD2 can be inhibited or suppressed by RNA interference of a B4GALNT1 gene.

In the embodiment of the present invention, the "immune checkpoint molecule" means a molecule that has an original function of suppressing an immune response to the molecule itself and also suppresses an excessive immune response, wherein this molecule is expressed in immune cells, such as, for example, T cells and B cells. In the embodiment of the present invention, the immune checkpoint molecule is defined to be a molecule expressed in T cells. The "immune checkpoint molecule" is not particularly limited, and examples thereof may include PD-1, CTLA-4, TIM-3, and LAG-3 (Lymphocyte Activation Gene-3).

In the embodiment of the present invention, the "T cell" means a cell that expresses a T-cell receptor (TCR) on the surface thereof. The T cell is not particularly limited, and examples thereof may include a CD8-positive T cell, a CD4-positive T cell, a suppressor T cell, a regulatory T cell (Treg cell), an effector T cell, a naive T cell, a memory T cell, an αβ T cell, and a γδ T cell. Moreover, , the T cell may also be CAT (CD3 Activated T Lymphocyte) or CAR-T (chimeric antigen receptor T) cell. Furthermore, the "T cell" used in the embodiment of the present invention also includes a progenitor cell thereof.

The T cell may be collected from a living body including, for example, blood such as peripheral blood or umbilical cord blood, or a body fluid such as a bone marrow fluid. Alternatively, the T cell may be induced to differentiate from pluripotent stem cells such as iPS cells.

UDP-glucose ceramide glucosyltransferase (UGCG, EC 2.4.1.80) is an enzyme that catalyzes a process of transferring glucose from UDP-glucose to ceramide. The amino acid sequence and cDNA sequence of human UDP-glucose ceramide glucosyltransferase are as set forth in SEQ ID NOS: 1 and 2, respectively. However, the "UDP-glucose ceramide glucosyltransferase" used in the embodiment of the present invention is not limited to human-derived UGCG, and it may also be derived from other mammals (which do not only include a mouse, a rat, a dog, and a cat, but also include livestock animals such as a bovine, a horse or sheep, and primates such as a monkey, a chimpanzee or a gorilla). Examples of the present UDP-glucose ceramide glucosyltransferase may include a protein comprising an amino acid sequence as set forth in SEQ ID NO: 1 and a protein comprising an amino acid sequence substantially identical to the amino acid sequence as set forth in SEQ ID NO: 1.

Herein, the "protein comprising an amino acid sequence substantially identical to the amino acid sequence as set forth in SEQ ID NO: 1" means a protein comprising an amino acid sequence having an amino acid identity of approximately 60% or more, preferably approximately 70% or more, more preferably approximately 80%, 81%, 82%, 83%, 84%, 85%, 86%, 87%, 88%, 89%, 90%, 91%, 92%, 93%, 94%, 95%, 96%, 97%, or 98%, and most preferably approximately 99%, with respect to the amino acid sequence as set forth in SEQ ID NO: 1, and having UDP-glucose ceramide glucosyltransferase activity.

Otherwise, the protein comprising an amino acid sequence substantially identical to the amino acid sequence as set forth in SEQ ID NO: 1 means a protein consisting of an amino acid sequence comprising a deletion, substitution, insertion or addition of one or several amino acids (preferably approximately 1 to 30, more preferably approximately 1 to 10, and further preferably 1 to 5 amino acids) in the amino acid sequence as set forth in SEQ ID NO: 1, and having UDP-glucose ceramide glucosyltransferase activity.

Moreover, the cDNA sequence encoding human UDP-glucose ceramide glucosyltransferase includes a cDNA encoding the above-described "protein comprising the amino acids sequence as set forth in SEQ ID NO: 1," as well as the nucleic acid as set forth in SEQ ID NO: 2.

In the embodiment of the present invention, the "method of reducing or losing the function of UDP-glucose ceramide glucosyltransferase (UGCG)" may include a method comprising allowing a UGCG activity inhibitor to come into contact with a T cell *ex vivo* to introduce the UGCG activity inhibitor into the T cell, and a method of suppressing the expression of a UGCG protein, or losing or reducing the enzyme activity of the expressed protein, by knocking out the UGCG gene of a T cell or introducing a mutation into the gene.

Examples of the UGCG activity inhibitor may include: 1-phenyldecanoylamino-3-morpholino-1-propanol (PDMP), 1-phenyl-2-hexadecanoylamino-3-morpholino-1-propanol (PPMP), butyl-deoxynojirimycin, and butyl-deoxygalactonojirimycin; and also, a neutralizing antibody against UGCG, and siRNA, miRNA and the like for knocking out the UGCG gene.

Knocking out of the UGCG gene can be carried out, for example, by genetic recombination using a cre/loxP system, etc., an RNAi method involving introduction of the aforementioned siRNA, miRNA, etc., and the like.

The first embodiment of the present invention also includes a T cell that is prepared by the method for producing or inducing a T cell of the present invention, wherein the function of UGCG or a ganglioside is reduced or lost and an increase in the expression of an immune checkpoint molecule (e.g. PD-1, TIM3, CTLA4, etc.) is not induced by T cell activation (i.e., the expression is suppressed) (hereinafter also referred to as "the T cell of the present invention"). Moreover, the T cell of the present invention also includes a T cell, in which the function of UGCG or a ganglioside is reduced or lost, the T cell being activated by TCR/CD3 stimulation or the like.

A second embodiment of the present invention relates to an immune checkpoint inhibitor comprising, as an active ingredient, a UDP-glucose ceramide glucosyltransferase (UGCG) inhibitor or an inhibitor of an enzyme catalyzing the biosynthesis of each ganglioside.

In the inhibitor according to the second embodiment of the present invention (hereinafter also referred to as "the inhibitor of the present invention"), the UDP-glucose ceramide glucosyltransferase inhibitor or the enzyme of catalyzing the biosynthesis of each ganglioside, used as an active ingredient, is desirably supported on a drug carrier that targets a T cell. As such a drug carrier, for example, a drug carrier, in which a molecule specifically targeting a T cell is allowed to bind to a nanocarrier used in DDS (drug delivery system), such as a liposome, a polymeric micelle or an albumin carrier, can be used.

As such a molecule specifically targeting a T cell, for example, a drug carrier, to which a molecule (an antibody, a peptide, an aptamer, etc.) specifically recognizing a T cell marker (e.g. a molecule specifically expressed on the surface of a T cell, such as TCR, CD3, CD4, CD8, or PD-1) is allowed to bind, can be used.

As mentioned above, the present inventors have found that, if the function of UGCG in a T cell is reduced or lost, even if the T cell is activated, the expression of an immune checkpoint molecule such as PD-1, TIM3 or CTLA4 is not increased (i.e., the expression of an immune checkpoint molecule associated with T cell activation is suppressed). Specifically, it is considered that, in a T cell treated *ex vivo* or *in vivo* with the UGCG inhibitor or the inhibitor of an enzyme catalyzing the biosynthesis of each ganglioside, the expression of an immune checkpoint molecule such as PD-1, TIM3 or CTLA4 associated with the T cell activation is suppressed, and that activation of the T cell is maintained.

Hence, a third embodiment of the present invention relates to an agent for suppressing the expression of PD-1 in a T cell, or an agent for maintaining the activation of a T cell, both of which comprise, as an active ingredient, a UGCG inhibitor or an inhibitor of an enzyme catalyzing the biosynthesis of each ganglioside.

A fourth embodiment of the present invention relates to a pharmaceutical composition for the prevention or treatment of a cancer, comprising the T cell of the present invention or the inhibitor of the present invention (hereinafter also referred to as "the pharmaceutical composition of the present invention").

The pharmaceutical composition of the present invention may be administered in the form of a pharmaceutical composition comprising an active ingredient (e.g. a UDP-glucose ceramide glucosyltransferase inhibitor, or a UDP-glucose ceramide glucosyltransferase inhibitor supported on a DDS carrier) and one or two or more pharmaceutical additives. Moreover, the pharmaceutical composition according to the present embodiment may also comprise known other drugs.

The pharmaceutical composition of the present invention may have a dosage form for oral or parenteral administration, and thus, the dosage form of the present pharmaceutical composition is not particularly limited. Examples of the dosage form of the present pharmaceutical composition may include a tablet, a capsule, a granule, a powder agent, a syrup, a suspending agent, a suppository, an ointment, a cream, a gelling agent, a patch, an inhalant, and an injection. These preparations are produced according to ordinary methods. Besides, in the case of a liquid preparation, the present pharmaceutical composition may be dissolved or suspended in water or another suitable solvent when it is used. In addition, in the case of a tablet or a granule, the present pharmaceutical composition may be coated according to a publicly known method. In the case of an injection, it is produced by dissolving the antibody of the present antibody or a functional fragment thereof in water. The present antibody or a functional fragment thereof may also be dissolved in a normal saline or a glucose solution, as necessary. Furthermore, a buffer or a preservative may be added to the mixed solution.

The types of pharmaceutical additives used in the production of the pharmaceutical composition of the present invention, the ratio of such pharmaceutical additives to the active ingredient, or a method for producing the pharmaceutical composition can be appropriately selected by a person skilled in the art, depending on the form thereof. As pharmaceutical additives, inorganic or organic substances, or solid or liquid substances can be used. In general, such pharmaceutical additives may be mixed into the present pharmaceutical composition in the range of, for example, 0.1% by weight to 99.9% by weight, 1% by weight to 95.0% by weight, or 1% by weight to 90.0% by weight, with respect to the weight of the active ingredient. Specific examples of the pharmaceutical additives may include lactose, glucose, mannit, dextrin, cyclodextrin, starch, sucrose, magnesium aluminometasilicate, synthetic aluminum silicate, sodium carboxymethyl cellulose, hydroxypropyl starch, calcium carboxymethyl cellulose, ion exchange resin, methyl cellulose, gelatin, gum Arabic, hydroxypropyl cellulose, hydroxypropylmethyl cellulose, polyvinyl pyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, nonionic surfactant, propylene glycol, and water.

To produce a solid preparation for use in oral administration, the active ingredient is mixed with an excipient component, such as, for example, lactose, starch, crystalline cellulose, calcium lactate or anhydrous silicic acid, to prepare a powder agent. Otherwise, as necessary, a binder such as white sugar, hydroxypropyl cellulose or polyvinyl pyrrolidone, a disintegrator such as carboxymethyl cellulose or calcium carboxymethyl cellulose, and other components are further added to the aforementioned mixture, and the thus obtained mixture is subjected to wet or dry granulation to prepare a granule. To produce a tablet, such a powder agent or a granule may be directly tableted, or after addition of a lubricant such as magnesium stearate or talc, the obtained mixture may be tableted. Such a granule or tablet may be coated with an enteric base material such as hydroxypropylmethyl cellulose phthalate or a methacrylic acid-methyl methacrylate polymer to prepare an enteric coated preparation, or may be coated with ethyl cellulose, carnauba wax, hardened oil or the like to prepare a sustained release preparation. In addition, to produce a capsule, a powder agent or a granule may be filled into a hard capsule, or the active ingredient may be directly coated with gelatin, or may be dissolved in glycerin, polyethylene glycol, sesame oil, olive oil or the like, and may be then coated with gelatin to prepare a soft capsule.

To produce an injection, the active ingredient may be dissolved in distilled water for injection, as necessary, together with a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and thereafter, the obtained solution may be subjected to aseptic filtration, and the resultant may be then filled into an ampoule. Otherwise, mannitol, dextrin, cyclodextrin, gelatin or the like may be further added to the resultant, followed by vacuum lyophilization, so that an injection that is soluble when used may be produced. Alternatively, lecithin, polysorbate 80, polyoxyethylene hardened castor oil or the like may be added to the active ingredient, and the obtained mixture is then emulsified in water, so that an emulsion for injection may also be produced.

To produce a rectal administration agent, the active ingredient may be dissolved by being humidified with a base material for suppository, such as cacao butter, fatty acid tri-, di- and mono-glyceride, or polyethylene glycol, and the obtained solution may be poured into a mold and may be then cooled. Otherwise, the active ingredient may be dissolved in polyethylene glycol, soybean oil or the like, and may be then coated with a gelatin film or the like.

The dose and the number of doses of the pharmaceutical composition of the present invention are not particularly limited, and can be selected, as appropriate, by doctor's or pharmacist's judgment, depending on conditions such as the purpose of prevention and/or treatment of deterioration and/or progression of a therapeutic target disease, the type of the disease, and the body weight and age of a patient.

In general, the daily dose per adult of the present pharmaceutical composition via oral administration is approximately 0.01 to 1,000 mg (the weight of the active ingredient), and it can be administered once a day or divided over several administrations, or every several days. In the case of using the present pharmaceutical composition as an injection, the pharmaceutical composition is desirably administered at a daily dose per adult of 0.001 to 100 mg (the weight of the active ingredient), continuously or intermittently.

A fifth embodiment of the present invention relates to a method for screening for an immune checkpoint-inhibiting substance, which comprises allowing a test substance to come into contact with a T cell, and then measuring the amount of Glc-Cer generated in the T cell or the expression level of UGCG in the T cell.

In the screening method according to the fifth embodiment of the present invention (hereinafter also referred to as "the screening method of the present invention"), purification and quantification of Glc-Cer can be easily carried out according to a known method, such as, for example, a method using thin-layer chromatography or HPLC. When the amount of Glc-Cer generated in a T cell with which a test substance has been allowed to come into contact is, for example, approximately 80% or less, preferably approximately 60% or less, more preferably approximately 40% or less, further preferably approximately 20% or less, and most preferably approximately 10% or less, compared with the amount of Glc-Cer generated in a control T cell with which the test substance has not been allowed to come into contact, it can be determined that the amount of Glc-Ger generated has been suppressed by the test substance, and this test substance can be considered to be a candidate of an immune checkpoint-inhibiting substance.

Moreover, in the screening method of the present invention, the expression level of UGCG can be measured according to a known method, for example, according to a PCR method such as an RT-PCR method or a real-time PCR method, a Northern blotting method, or a high-throughput assay method using a microarray or the like. When the expression level of UGCG in a T cell with which the test substance has been allowed to come into contact is, for example, approximately 80% or less, preferably approximately 60% or less, more preferably approximately 40% or less, further preferably approximately 20% or less, and most preferably approximately 10% or less, compared with the expression level of UGCG in a control T cell with which the test substance has not been allowed to come into contact, it can be determined that the expression level of UGCG has been suppressed by the test substance, and this test substance can be considered to be a candidate of an immune checkpoint-inhibiting substance. The immune checkpoint-inhibiting substance can also become a candidate of a preventive or therapeutic agent for cancer, the screening method of the present invention can also be a method for screening for a candidate of a preventive or therapeutic agent for cancer.

A sixth embodiment of the present inventio relates to a method for preventing and/or treating a cancer, which comprises administering the pharmaceutical composition of the present invention or the T cell of the present invention to a patient (hereinafter also referred to as "the preventive or therapeutic method of the present invention").

Herein, the term "treatment" means to stop or alleviate the progression and deterioration of a pathological condition in a patient who has already been affected with a cancer, and it is a treatment performed for purpose of stopping or alleviating the progression and deterioration of the cancer.

On the other hand, the term "prevention" means to stop, in advance, the onset of a cancer to be treated in a subject who is likely to develop the cancer, and it is a treatment performed for the purpose of stopping the onset of a cancer in advance. Moreover, a treatment performed to stop the recurrence of a cancer after completion of the cancer therapy is also included in the "prevention."

Furthermore, therapeutic and preventive targets are not limited to humans, and may also be mammals other than humans, such as, for example, mice, rats, dogs or cats, livestock animals such as bovines, horses or sheep, and primates such as monkeys, chimpanzees or gorillas. The therapeutic and preventive targets are particularly preferably humans.

In the method of administering the above-described T cell of the present invention to a patient, the T cell may be the T cell of the present invention that is produced from CAT or a CAR-T cell derived from the patient him/herself or a donor (another person), or may also be the T cell of the present invention, in which CD3 is activated, or a CAR-T cell produced from the T cell of the present invention.

Cancers as targets of the preventive or therapeutic method of the present invention may include malignant tumors and neoplasms.

Examples of the malignant tumor may include hepatocellular carcinoma, cholangiocarcinoma, renal cell carcinoma, squamous cell carcinoma, basal cell carcinoma, transitional cell carcinoma, adenocarcinoma, malignant gastrinoma, malignant melanoma, fibrosarcoma, mucinous sarcoma, liposarcoma, leiomyosarcoma, rhabdomyosarcoma, malignant teratoma, angiosarcoma, Kaposi's sarcoma, osteosarcoma, chondrosarcoma, lymphangioma, malignant meningioma, non-Hodgkin's lymphoma, Hodgkin's lymphoma, leukemia, and brain tumor.

Examples of the neoplasm may include epithelial cell-derived neoplasm (epithelial carcinoma), basal cell carcinoma, adenocarcinoma, lip cancer, oral cancer, esophageal cancer, gastrointestinal cancers such as small intestine cancer and stomach cancer, colon cancer, rectal cancer, liver cancer, bladder cancer, pancreatic cancer, ovarian cancer, cervical cancer, lung cancer, breast cancer, skin cancers such as squamous epithelial cell carcinoma and basal cell carcinoma, prostate cancer, and renal cell carcinoma. In addition, examples of the neoplasm may also include known other cancers that affect the epithelium, mesenchyme or blood cells in a whole body.

When an English translation of the present description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Analysis using T cell-specific UGCG gene-deficient mice

Malignant melanoma was transplanted into T cell-specific UGCG gene-deficient mice, and the growth of the tumor and the survival period of the mice were then examined. Besides, in the experiments using mice, which were performed in the present Examples, all of the mice were fed under an SPF (specific pathogen free) environment, and were treated in accordance with the guidelines under the approval of the Animal Experiment Expert Committee, Azabu University of Tokyo.

The T cell-specific UGCG gene-deficient mice were produced by crossing UGCG gene-deficient flox mice (Yamashita et al., Genesis 43: 175-180 2005) with T cell-specific Cre mice. In addition, littermate mice, which did not have a cre gene, were used as controls.

1 × 10⁵ Murine melanoma cells (B16F110: obtained from the Cell Resource Center for Biomedical Research, Cell Bank, Tohoku University) were suspended in 100 µl of Dulbecco's modified Eagle medium (DMEM) supplemented with 30% Matrigel (CORNING), and the obtained suspension was then injected into the dorsal subcutis of the T cell-specific UGCG gene-deficient mice and the control mice. After transplantation of the melanoma cells, the mice were euthanized over time, the tumor was then excised therefrom, and the volume thereof was then measured. It was found that the growth of the tumor of the melanoma cells that had been transplanted into the T cell-specific UGCG gene-deficient mice was retarded (Figure 2).

Subsequently, a Kaplan-Meier survival curve was obtained to compare the survival rate of the T cell-specific UGCG gene-deficient mice and that of the control mice, and a log-rank test was carried out. Regarding the mice into which the melanoma cells had been transplanted, a case where the mice perished, a case where the tumor volume exceeded 1500 mm³, a case where apparent respiratory abnormalities were found, a case where the body weight was reduced by 10% or more, and a case where apparent metastasis was found by visual observation, were defined as endpoints, and the analysis of the survival rate was carried out. As a result, the effect of extending the life for at least about 1 week was found in the T cell-specific UGCG gene-deficient mice (Figure 3).

The tumor tissues of each mouse were fixed with formalin, and were then embedded in paraffin, followed by HE staining. In the tissues derived from the T cell-specific UGCG gene-deficient mice, accumulation of lymphocytes was observed (Figure 4A, right). Moreover, the T cells shown in Figure 4A were counted by visual observation. As a result, the T cells in the tumor of the T cell-specific UGCG gene-deficient mouse tended to increase (Figure 4B). Accordingly, it was suggested that the T cells actively attacked the tumor.

### 2. Configuration of T cells in spleen of T cell-specific UGCG gene-deficient mice

The T cell-specific UGCG gene-deficient mice and the control mice were fed for 14 days under melanoma cell transplanted conditions or under melanoma cell nontransplanted conditions, and thereafter, the spleen was excised from each mouse. Using Dynabeads Untouched Mouse T Cells kit (Thermo fisher Scientific), T cells were isolated from the excised spleen.

First, the amount of gangliosides present in the isolated T cells was examined. The T cells were stained with an anti-CD3 antibody (T cell marker) and an FITClabeled cholera toxin (that binds to the gangliosides), and were then analyzed using a flow cytometer (EC800, Sony). As a result, it was confirmed that the amount of gangliosides was reduced in the T cells in the splenic cells of the T cell-specific UGCG gene-deficient mice, and thus that the biosynthesis of many gangliosides located downstream of UGCG was inhibited (Figure 5).

Subsequently, the T cells were subjected to multiple staining with an anti-CD3 antibody, an anti-CD4 antibody and an anti-CD8 antibody, and were then analyzed using a flow cytometer (Figure 6). As a result, it was found that the total number of T cells in the spleen of wild-type mice (WT) was reduced after transplantation of the melanoma cells (Figure 6, left), and that, in particular, a reduction in CD8⁺ T cells was significant (Figure 6, right). Thus, it was confirmed that the T cells were exhausted by the tumor. In the T cell-specific UGCG gene-deficient mice (KO), the CD4⁺ cells and the CD8⁺ cells increased after transplantation of the tumor, and in particular, an increase in the CD8⁺ cells was significant (Figure 6, center and right). Accordingly, it was confirmed that the T cell-specific UGCG gene-deficient mice acquired a trait by which T cells were hardly exhausted.

### 3. Analysis of expression status of immune checkpoint molecule after activation of T cells derived from T cell-specific UGCG gene-deficient mice

Using Dynabeads Untouched Mouse T Cells kit (Thermo fisher Scientific), T cells were isolated from the excised spleen, and the T cells were then activated by IL-2 (30 U/mL) and Dynabeads Mouse T-Cell activator CD3-CD28 (Thermo fisher Scientific). Thereafter, the T cells were recovered over time, and the expression of a PD-1 gene was then analyzed by qPCR. With regard to the proliferation rate of the T cells after the activation, the proliferation rate was increased in the cells derived from the T cell-specific UGCG gene-deficient mice (Figure 7A). Further, it is noteworthy that the expression level of PD-1 was clearly increased in the T cells derived from the control mice after activation of the T cells, whereas such an increase in the expression level of PD-1 was not observed in the T cells derived from the T cell-specific UGCG gene-deficient mice (Figure 7B).

From the aforementioned results, it became clear that the expression level of a PD-1 gene that is one of immune checkpoint molecules is hardly increased in UGCG gene-deficient T cells after activation of the T cells.

### 4. Analysis of expression status of immune checkpoint molecules after activation of CD4-positive T cells derived from T cell-specific UGCG gene-deficient mice

Using MojoSort™ Mouse CD4 T Cell Isolation Kit (BioLegend), CD4-positive T cells were isolated from the excised spleen, and the CD4-positive T cells were then activated in the same manner as that of the above 2. Thereafter, the T cells were recovered over time, and the expression of IFNy and PD-1 genes was then analyzed by qPCR.

As a result, it was found that the expression of IFNy was increased in the CD4-positive T cells derived from T cell-specific UGCG gene-deficient mice at an expression level equivalent to or greater than that in the T cells derived from wild-type mice, but that the expression of the immune checkpoint molecules PD-1, TIM3 and CTLA4 was decreased (Figure 8).

### 5. Measurement of proliferation rate of T cells derived from T cell-specific UGCG gene-deficient mice after activation thereof

The isolated T cells were activated in the same manner as that of the above 3, and the number of the T cells was then counted over time, using a hemocytometer. As a result, it could be confirmed that there was no difference in the proliferation rate of the activated T cells between the wild-type (WT) mice and the UGCG gene-deficient (KO) mice (Figure 9), and thus that a deficiency of the UGCG gene did not influence on the growth of the T cells.

### 6. Influence of in vitro PPMP treatment on glycolipids in T cells

The T cells were isolated in the same manner as that of the above 3, and the isolated T cells were then treated with PPMP used as a UGCG inhibitor in various concentrations for 24 hours. Thereafter, the expression level of ganglioside GM3 was analyzed using a flow cytometer. As a result, it could be confirmed that the expression level of GM3 was reduced in a PPMP concentration-dependent manner (Figure 10, left). Moreover, in order to examine the toxicity of the PPMP treatment on T cells, the proliferation rate of the T cells was measured using a cell proliferation/cytotoxicity assay kit (CK-1, Dojindo). As a result, it was suggested that the PPMP treatment, at least, in a concentration range of reducing glycolipids, did not influence on the proliferation rate, and thus that the toxicity of PPMP on the cells was low (Figure 10, right).

### Industrial Applicability

According to the present invention, a T cell in which the expression of an immune checkpoint molecule is not induced, an immune checkpoint inhibitor, a UGCG inhibitor, and the like are provided, and these are used in the prevention or treatment of cancer. Therefore, it is expected that the present invention will be utilized in the medical field.

## Claims

1. A method for producing or inducing a T cell in which the expression of an immune checkpoint molecule is not induced, the method comprising reducing or losing the function of UDP-glucose ceramide glucosyltransferase (UGCG) or ganglioside in a T cell.

2. The method according to claim 1, which is **characterized in that** the immune checkpoint molecule is PD-1 (Programmed Death-1), TIM3 (T-cell Immunoglobulin and Mucin domain 3), or CTLA4 (Cytotoxic T-Lymphocyte Associated antigen 4).

3. The method according to claim 1 or 2, wherein the method of reducing or losing the function of the UGCG is a reduction in the expression of the UGCG gene or a loss thereof.

4. The method according to claim 1 or 2, which is **characterized in that** the method of reducing or losing the function of the UGCG is a treatment with a UDP-glucose ceramide glucosyltransferase inhibitor.

5. A T cell comprising UGCG whose function is reduced or lost, in which an increase in the expression of an immune checkpoint molecule by activation is not induced.

6. An immune checkpoint inhibitor comprising a UGCG inhibitor as an active ingredient.

7. The inhibitor according to claim 6, which is **characterized in that** it is supported on a drug carrier that targets a T cell.

8. A pharmaceutical composition for the prevention or treatment of a cancer, comprising the T cell according to claim 5 or the inhibitor according to claim 6 or 7.

9. An agent for suppressing the expression of PD-1, TIM3, and/or CTLA4 in a T cell, the agent comprising a UGCG inhibitor as an active ingredient.

10. An agent for maintaining the activation of a T cell, the agent comprising a UGCG inhibitor as an active ingredient.

11. A method for screening for an immune checkpoint-inhibiting substance, comprising allowing a test substance to come into contact with a T cell, and then measuring the amount of Glc-Cer generated in the T cell.

12. A method for screening for an immune checkpoint-inhibiting substance, comprising allowing a test substance to come into contact with a T cell, and then measuring the expression level of UGCG in the T cell.

13. The screening method according to claim 11 or claim 12, wherein the immune checkpoint-inhibiting substance is a preventive or therapeutic agent for cancer.
